(19)

**Europäisches Patentamt**
**European Patent Office**
**Office européen des brevets**

(11) **EP 4 621 411 A1**

(12) **EUROPEAN PATENT APPLICATION**

(43) Date of publication:
24.09.2025 Bulletin 2025/39

(21) Application number: 24382317.6

(22) Date of filing: **22.03.2024**

(51) International Patent Classification (IPC):
*G01N 33/68* *(2006.01)*

(52) Cooperative Patent Classification (CPC):
**G01N 33/6854;** G01N 2800/24

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Leti Pharma S.L.**
**08038 Barcelona (ES)**

(72) Inventors:
• **ÁLVAREZ ÁLVAREZ, Javier**
**08038 Barcelona (ES)**
• **PARODY DE LA FUENTE, Nuria**
**08038 Barcelona (ES)**
• **CARNÉS SÁNCHEZ, Jerónimo**
**08038 Barcelona (ES)**

(74) Representative: **Stratagem IPM Limited**
**Meridian Court**
**Comberton Road**
**Toft, Cambridge CB23 2RY (GB)**

(54) **METHOD FOR DETECTING SIALYLATED IGE**

(57)   The invention relates to a method of quantifying sialylated IgE in a test sample, the method comprising: immobilising sialylated IgE in the test sample via a capture probe which is specific for sialylated glycoproteins, wherein the capture probe is immobilised; and using an antibody-based detection technique to quantify sialylated IgE. The invention further relates to: diagnosis of allergy in a subject, methods of treatment comprising diagnosis of allergy followed by treatment by immunotherapy, kits comprising reagents for the method, and use of the kits to quantify sialylated IgE in a test sample.

**EP 4 621 411 A1**

## Description

The present invention relates to methods and kits for the quantification of sialylated IgE in test samples which may be derived from human or animal subjects. Methods of the invention may be used as part of diagnosis of allergy or allergic disease.

## Background

Allergy is caused by hypersensitivity of the immune system to different substances (allergens) present in the environment. Allergy can manifest clinically as an allergic disease. Such diseases include food allergy, asthma, atopic dermatitis, allergic rhinitis, conjunctivitis, and chronic rhinosinusitis.

Allergic sensitisation is the process by which a person develops significant levels of specific IgE antibodies (sIgE) against an allergen through repeated exposure as a consequence of a deviation of the immune system.

Allergy diagnosis is a complex and multistep process. It begins with a clinical interview to elaborate the patient's history, and a physical examination. Additional tests are used to identify the allergenic source/s that is/are related with symptoms through detection of sIgE. Two type of tests can be performed, including *in vivo* and *in vitro*. *In vivo* tests (i.e. skin prick tests, intradermal test, etc...) are based on the demonstration of allergen sIgE by the direct exposure of the skin to allergens and measuring of the wheal size produced within 30 minutes after allergen application. *In vitro* tests detect circulating sIgE antibodies in serum using laboratory methods (ImmunoCAP™, ALEX2, etc) (1).

Frequently, practitioners initially use *in vivo* testing and, in the case of a positive response, confirm the reaction to the positive allergens by *in vitro* methods. Confirmation of allergen sensitisation and the identification of causal allergens are essentials for optimising the management of allergic conditions. However, it is known that many subjects have positive skin tests and/or positive serum sIgE without allergic symptoms because a positive allergy test result indicates only sensitisation. It does not necessarily mean clinical allergy (1). Moreover, the concentration of sIgE determined by *in vitro* tests and/or the value of the response obtained by skin tests does not usually correlate with the severity of symptoms (1, 2). Another limitation of sIgE tests, especially *in vivo,* is that they can produce false negatives (negative result in patients with allergic disease). This can be critical when allergic symptoms are associated with life-threatening anaphylaxis in subjects with allergy to foods or Hymenoptera venoms (1).

Apart from sIgE, the measurement of total IgE in sera was considered in early studies as the simplest way to identify subjects with allergy but it soon became evident that total IgE levels could not be considered a reliable marker of allergy status. Total IgE levels significantly higher than the normal threshold are usually associated with allergic disorders, but also with other conditions. Furthermore, low or normal total IgE levels do not exclude the presence of IgE-mediated diseases. Therefore, levels of total IgE should be carefully interpreted and not considered as an indication for the presence of allergy (1).

Thus, there is currently a lack of clinically validated *in vitro* methods that can be used as complementary tools for the diagnosis of allergy and allergic diseases.

IgE is the most glycosylated immunoglobulin in human and possesses seven potential N-glycosylation sites within the constant region of the heavy chain located in the asparagine (N) residues: N21, N49, N99, N146, N252, N264 and N275 (Uniprot P01854) (3). Site-mutagenesis studies (3, 4) have established that:

N275-linked oligomannose is required for appropriate IgE folding and binding to receptors to initiate effector functions
N264 is unoccupied; and

The remaining five sites are occupied by complex antennary glycans.

Glycosylation studies performed in samples from allergic patients and non-allergic subjects determined that 5-acetylneuroaminic acid (sialic acid) residues were enriched in the terminal position of antennary glycans of positions N49 and N146 in patients with allergy. These sialic acid moieties are linked to galactose by $\alpha$-2,6 bridges. Enzymatic removal of sialic acid from IgE produced a statistically significant reduction of the allergenic activity of the IgE from allergic mice and in *in vitro* human model experiments (5). These results demonstrated that specific sialylation patterns can distinguish the IgE of subjects with allergy from non-allergic subjects and thus can act as an important biomarker for the pathogenicity of IgE and the diagnosis of allergy.

Current methods for detecting the sialylation of proteins, including IgE, are laborious using techniques such as enzymatic digestion followed by liquid chromatography with tandem mass spectrometry (5). This method is challenging to employ in a clinical setting because IgE is the least abundant immunoglobulin in blood and therefore large volumes of blood are required to prepare enough material to test. The IgE must be effectively pure which requires a multistep purification process. This method requires highly skilled laboratory workers, access to expensive machinery and complex analysis. Therefore, there remains a need for a simple technique to quantify sialylation of IgE which requires minimal sample processing and produces a readily interpretable readout.

A technique developed by Wu et al. for quantifying the sialylation of the IgG family of immunoglobulins requires minimal sample processing and provides a spectroscopic readout. This method utilises the interaction between sialic acid and SNA-1 lectin to specifically immobilise sialylated IgG from sera. IgG-specific horseradish peroxidase (HRP) conjugate antibodies were then used to colorimetrically detect the bound, sialylated IgG through the activity of HRP (7).

The present invention comprises the successful adaptation of the technique of Wu et al. for the quantification of sialylated IgE and the correlation of this parameter with allergic disease. The success of this technique is surprising given the relative scarcity of IgE in sera when compared to, for example, IgG (3). One would expect that other, more abundant sialylated proteins would mask any differences in the sialylation status of IgE.

**Summary of the invention**

In a first aspect of the invention, a method of quantifying sialylated IgE in a test sample is provided, the method comprising:

immobilising sialylated IgE in the test sample via a capture probe which is specific for sialylated glycoproteins, wherein the capture probe is immobilised; and
using an antibody-based detection technique to quantify sialylated IgE.

In a second aspect of the invention, a diagnostic method for diagnosing allergy in a subject is provided, the diagnostic method comprising using the method of the first aspect of the invention and diagnosing the subject with allergy based on the quantity of sialylated IgE in the test sample.

In a third aspect of the invention, a method of treatment of allergy or allergic disease is provided, the method comprising the steps of:

diagnosis of allergy by the diagnostic method according to the second aspect of the invention; and
treatment by immunotherapy.

In a fourth aspect of the invention, a kit is provided, the kit comprising:

a capture probe which is specific for sialylated glycoproteins wherein the capture probe is either immobilised or is readily capable of immobilisation; and
an IgE recognising antibody.

In a fifth aspect of the invention, use of the kit of the fifth aspect of the invention is provided to quantify sialylated IgE in a test sample.

**Figures**

The invention is now exemplified with reference to:

Figure 1 which shows analysis of serial dilutions of purified myeloma IgE and Bovine Serum Albumin (BSA) from 6.4 pg/ml to 50 $\mu$g/ml. Results are expressed in ELISA Arbitrary Units (EAU) which are equivalent to $A_{450\ nm}$;

Figure 2 which shows analysis of serial dilutions of sera from human subjects with allergic disease (A, B and C) and one non-allergic (healthy) control (the sera were serial diluted from 1/5 to 1/640; healthy control had 0.00 kU/L (8) of total IgE; results are expressed in EAU which are equivalent to $A_{450\ nm}$;

Figure 3 which shows a box-plot representation of results from human subjects with allergic disease (n = 405) and non-allergic human subjects (n = 30) (values are expressed in EAU which are equivalent to $A_{450\ nm}$; **** shows statistical differences between groups (p < 0.0001). Filled circles in the allergic group correspond to results from patients sensitized to venoms;

Figure 4 which shows a ROC curve generated by the analysis of sera from the same 30 non-allergic (controls) human subjects and 405 human subjects with allergic disease of Figure 3;

Figure 5 which shows correlation analysis between total IgE (kU/L) (8) and Sialic-IgE ELISA (EAU) of sera from human subjects with allergic disease (n = 404);

Figure 6 which shows the results of the analysis of levels of sialylation of IgE by an alternative method in sera from 3 subjects suffering from allergic disease and 3 non-allergic controls (the sera were serial diluted from 1/50 to 1/12150 with results expressed as $A_{415\ nm}$);

Figure 7a which shows a schematic of the method according to the invention (see Example 2);

Figure 7b which shows a schematic of an alternative method which is not part of the invention (see Example 8).

**Detailed description of the invention**

In a first aspect of the invention, a method of quantifying sialylated IgE in a test sample is provided, the method comprising:

immobilising sialylated IgE in the test sample via a capture probe which is specific for sialylated glycoproteins, wherein the capture probe is immobilised; and
using an antibody-based detection technique to quantify sialylated IgE.

Sialylated IgE means an immunoglobulin from the IgE family which comprises at least one glycan further comprising a sialic acid moiety. IgE has different glycosylation sites and sialylated IgE may only comprise a single sialic acid moiety or can comprise up to 30 sialic acid moieties. Multiple sialic acid moieties may be part of the same glycan and therefore be connected to the same residue and/or may be spread over multiple glycans.

Immobilising sialylated IgE via a capture probe means binding sialylated IgE to a surface such that the remaining test sample can be removed whilst leaving the bound sialylated IgE behind, thus separating the sialylated IgE from other components of the test sample. This can be achieved by several methods known to the skilled person. The surface is a macrostructure able to bind the capture probe. This binding may be achieved through functionalisation of the surface. The surface may be a solid surface. The surface may be selected from the well of a plate or a portion thereof, a bead, a membrane, a gel or other matrix. The surface enables bound material to be retained whilst unbound material can be readily washed away.

The capture probe may be any agent able to bind sialylated glycoproteins and also bind to a surface. This includes antibodies which recognise sialylated glycoproteins, lectins which recognise sialylated glycoproteins, other proteins which recognise sialylated glycoproteins and chemical agents which recognise sialylated glycoproteins. The capture probe may bind to a surface through a covalent interaction such as an N-succinamide linkage or isopeptide bond, or a non-covalent interaction such as a specific protein interaction (e.g. biotin/streptavidin), a chelating group, hydrogen bonding, hydrophobic interactions or a combination thereof. The corresponding surface should be functionalised accordingly to facilitate or enable the binding.

Quantifying sialylated IgE means measuring the amount or concentration of sialylated IgE in a test sample. Suitable antibody-based detection techniques utilise antibodies which are specific for an epitope of the bound sialylated IgE. Quantification can produce a relative quantity or an absolute quantity. An absolute quantity may be obtained by comparing the direct result of a detection technique with the results of a standard curve to infer the absolute quantity (in mass units, moles, etc.) of sialylated IgE in the test sample. The standard curve can be prepared by performing the method of the invention on samples of known concentration of sialylated IgE. A relative quantity is not linked to a physical unit of quantity but is interpreted directly from the results of the detection technique. A relative quantity may have units of absorbance or fluorescence counts, for example.

In an embodiment, the capture probe is *Sambucus nigra* agglutinin 1 lectin (SNA-1 lectin). This tetrameric protein recognises specifically the $\alpha$-2,6 link between galactose and sialic acid in glycans.

In an embodiment, the capture probe is immobilised to a solid surface. The solid surface may be the surface of a well of a plate or a bead, for example a magnetic bead, or a membrane, for example a nitrocellulose membrane. Preferably the solid surface is the surface of the well of an ELISA plate.

In an embodiment, the antibody-based detection technique comprises a colorimetric assay. Colorimetric assays measure the generation (or depletion) of a chromophore in a solution due to the presence (or absence) of a particular entity. Changes to the concentration of the chromophore change the absorbance (OD) of the solution according to the Beer-Lambert law. Measurement of absorbance of the solution can be readily carried out using a spectrophotometer which may be integrated into a plate reader for high-throughput assays.

In an embodiment, the antibody-based detection technique comprises the use of an IgE recognising antibody. The IgE recognising antibody is any antibody which specifically recognises the IgE being tested in the method. The IgE recognising antibody may be a monoclonal antibody. The IgE recognising antibody may be derived from mouse cells. Where the sialylated IgE being quantified has come from a human, the IgE recognising antibody may be obtained by immunizing a non-human animal (for example a mouse) with human IgE, isolating specific B-lymphocytes which produce the anti-human IgE antibodies from the non-human animal, fusing the B-lymphocytes with immortal myeloma cell lines to form hybridoma cell lines which produce anti-human IgE monoclonal antibodies. Where the sialylated IgE being quantified has come from a non-human animal, the IgE recognising antibody may be similarly obtained using a different species of non-human animal (rabbit, mouse, rat, dog, goat, pig etc.) to generate hybridomas.

In an embodiment, the IgE recognising antibody is conjugated to a horseradish peroxidase enzyme (HRP). HRP, in the presence of a suitable oxidant such as hydrogen peroxide, catalyses the oxidation of some organic compounds, such as diaminobenzidine (DAB), tetramethylbenzidine (TMB), and 2,2'-azino-bis(3-ethylbenzothiazoline-6-sulfonic acid (ABTS), to form chromophores which can be detected and quantified using a spectrophotometer. Compounds which can be oxidised by HRP in this manner are termed chromophore precursors. HRP can also catalyse the oxidation of luminol resulting in a chemiluminescent compound which can be detected using a fluorimeter.

In an embodiment, the method comprises:

a) immobilizing SNA-1 lectin in the well of an ELISA plate;
b) adding a test sample and allowing any sialylated glycoproteins to bind to the immobilised SNA-1 lectin;
c) adding an anti-IgE antibody conjugated to horseradish peroxidase and allowing the antibody to bind to any bound sialylated IgE; and
d) adding a developing solution comprising a chromophore precursor and hydrogen peroxide, allowing oxidation of the chromophore precursor to a chromophore to occur, and detecting the production of the chromophore using spectrophotometry.

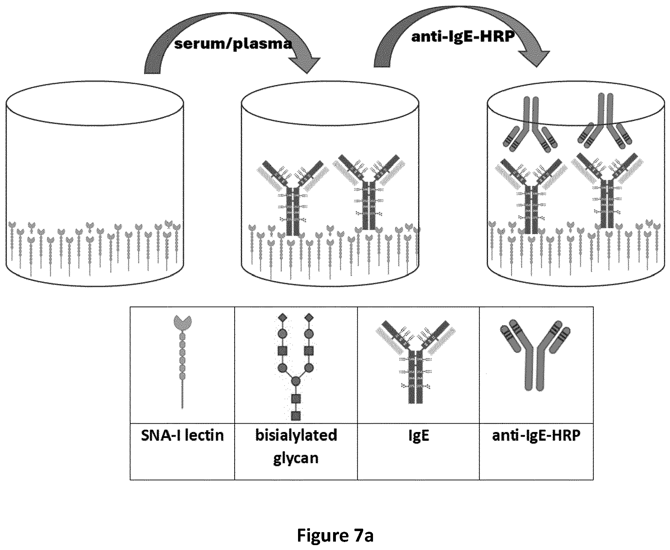

A schematic of this method is shown in Figure 7a.

The method may further comprise a blocking step between steps a) and step b) comprising the addition of an immunochemically inert substance to saturate the ELISA plate. The immunologically inert substance should interact neither with the SNA-1 lectin, the anti-IgE antibody or the conjugated horseradish peroxidase. The immunologically inert substance may be bovine serum albumin (BSA). Saturation of the plate means that any "vacant" binding sites are filled. This means that only the intended components of the test sample will be immobilised in the well via interaction with SNA-1 lectin.

The method may further comprise one or more washing steps between any previously disclosed step to remove any unbound material.

In an embodiment the chromophore precursor is diaminobenzidine.

In an embodiment, the test sample is blood serum or plasma from a subject. Serum is prepared from whole blood by allowing the blood to clot and then removing the clotted material by, for example, centrifugation. Plasma is prepared by obtaining blood from the subject, adding an anticoagulant to prevent clotting and removing the blood cells by, for example, centrifugation. The main difference between serum and plasma is that serum does not comprise clotting factors such as fibrinogen. It will be appreciated that both blood serum and plasma are only available *ex vivo* and thus are not part of a human or animal body.

The test sample may be diluted in a suitable buffer before the method of the invention is carried out. In this case the dilution factor should be considered in order to quantify the sialylated IgE present in the original test sample.

In an embodiment, the subject is a human subject. In an alternative embodiment, the subject is a non-human animal subject.

In a second aspect of the invention, a diagnostic method for diagnosing allergy in a subject is provided, the diagnostic method comprising using the method of the first aspect of the invention, wherein the test sample is blood serum or plasma from the subject, and determining from the quantity of sialylated IgE whether the subject suffers from allergy.

In an embodiment, where the test sample is blood serum from a human subject, the subject is diagnosed with allergy if the test sample comprises sufficient sialylated IgE such that, when tested according to a method comprising:

a) immobilizing SNA-1 lectin in a well of a standard 96-well plate;
b) diluting a test sample 20-fold into an inert buffer (preferably phosphate buffered saline);
c) adding 100 μL of the diluted test sample to the well and allowing any sialylated glycoproteins to bind to the immobilised SNA-1 lectin;
d) adding an anti-IgE antibody conjugated to horseradish peroxidase and allowing the antibody to bind to any bound sialylated IgE; and
e) adding a developing solution comprising diaminobenzidine and hydrogen peroxide, allowing oxidation of the chromophore precursor to a chromophore to occur,
f) halting oxidation though addition of a peroxidase inhibitor such that the total solution volume is 200 μL; and
g) detecting the production of the chromophore using spectrophotometry;

the chromophore elicits an absorbance of at least 0.55 at 450 nm.

A blocking step and/or washing steps as defined above may also be incorporated into the method. Each step may comprise an incubation period to allow binding to occur.

The maximum absorbance elicited by the chromophore may be the detection limit of the spectrophotometer which may optionally be 2.5, 2, 3, 3.5 or 4.

In an embodiment, if the subject is diagnosed with allergy, the diagnostic method further comprises a suitable clinical or *in vitro* method to determine if a subject is allergic to any allergy source. Suitable clinical or *in vitro* methods include those already known to the skilled person for diagnosing allergy, for example skin tests and methods to detect circulating sIgE antibodies in serum.

In a third aspect of the invention, a method of treatment of allergy or allergic disease is provided, the method comprising the steps of:

> diagnosis of allergy by the second aspect of the invention; and
> treatment by immunotherapy.

Immunotherapy involves the administration of increasingly larger doses of an allergen extract with the aim of inducing immunological tolerance. Allergen immunotherapy modulates the immune response to the allergen rather than ameliorating the symptoms induced by an allergic reaction, and can either reduce the need for medication, reduce the severity of symptoms or eliminate hypersensitivity altogether.

In a fourth aspect of the invention, a kit is provided, the kit comprising:

> a capture probe which is specific for sialylated glycoproteins wherein the capture probe is either immobilised or is readily capable of immobilisation; and
> an IgE recognising antibody.

Readily capable of immobilisation means that the capture probe does not need to be modified in order to be immobilised. For example, it may comprise a tag group which is intended to interact with a functionalised surface. The capture probe may be inherently readily capable of immobilisation due to its hydrophobic/hydrophilic properties.

In an embodiment, the capture probe is SNA-1 lectin.

In an embodiment, the IgE recognising antibody is conjugated to horseradish peroxidase.

In a fifth aspect of the invention, use of the kit according to the fourth aspect of the invention to quantify sialylated IgE in a test sample is provided.

**Examples**

**Example 1** - **Preparation of ELISA plates for the method of quantifying sialylated IgE**

A Maxisorp™ 96 well ELISA plate (Thermo Scientific™) was coated with SNA-1 (Vector laboratories) by adding 100 $\mu$l/well SNA-1 at 20 $\mu$g/ml in 0.01 M PBS (phosphate buffer solution) and incubating overnight.

The plate was washed with Buffer A (0.1 % (w/v) Tween™ 20 in 0.01 M PBS) and then blocked for 1 hour using 100 $\mu$l/well Buffer B (3% (w/v) BSA, 0.1 % (w/v) Tween™ 20 in 0.01 M PBS). The plate was then washed with Buffer A again.

**Example 2 - General method for quantifying sialylated IgE**

Plates were prepared according to Example 1.

Samples were prepared in Buffer A and added to the plates at 100 $\mu$l/well. Plates were incubated for 2 hours and then washed with Buffer A.

Mouse anti-human IgE monoclonal antibody conjugated to horseradish peroxidase (clone B3102E8, Southern Biotech) was diluted 500-fold in Buffer C (1 % (w/v) BSA, 0.1 % (w/v) Tween™ 20 in 0.01 M PBS) and 100 $\mu$L/well was added. The plates were incubated for 1 hour and then washed with Buffer A.

100 $\mu$L/well peroxidase substrate (k-Blue® TMB; Neogen) was added and the plates incubated for 20 minutes. The oxidation reaction was then stopped by adding 100 $\mu$l/well of concentrated $H_2SO_4$.

Absorbance of each well at 450 nm was measured using a plate reader.

A schematic of this method is shown in Figure 7a.

**Example 3 - Establishing working range of method for quantifying sialylated IgE**

Samples of myeloma purified IgE (Merck) from 6.4 pg/mL to 50 $\mu$g/mL as well as equivalent concentrations of BSA were prepared in Buffer A. Samples were added in triplicate to plates and the method of Example 2 was carried out.

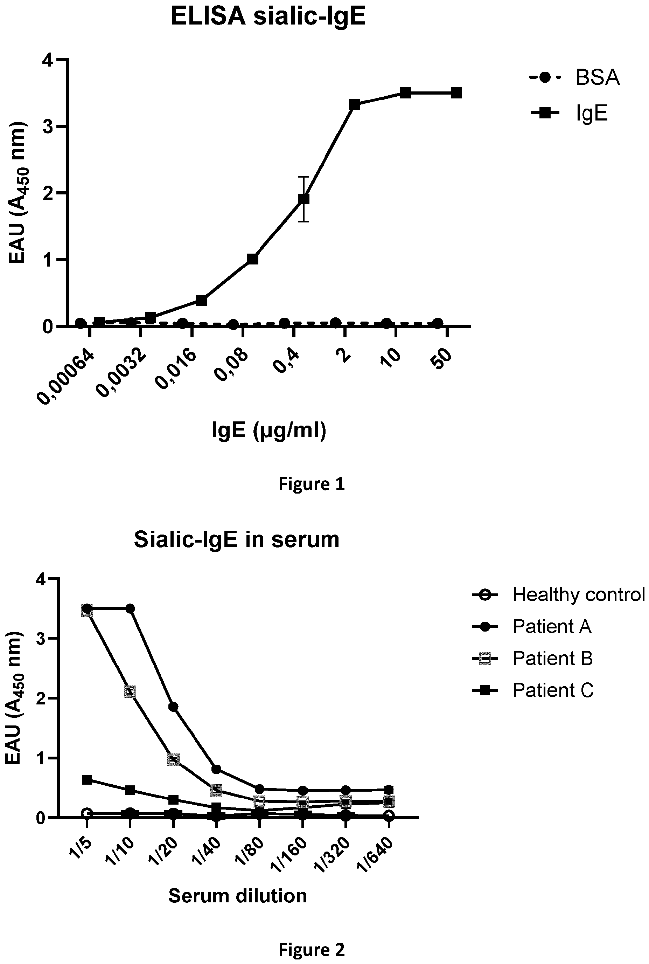

The results are shown in Figure 1 and demonstrate that the method can detect sialylated IgE and the method has a linear working range of between 80 ng/mL and 2 μg/mL myeloma purified IgE. The method is specific for purified IgE as evidenced by the results for the BSA control samples.

**Example 4 - Detection of sialylated IgE in serum**

Sera from three allergic subjects (A, B and C) and one healthy patient without allergic disease were obtained. Total IgE concentration as determined by ImmunoCAP™ assay is shown in Table 1.

Table 1: Total IgE for the sera for subjects A-C and a healthy subject

| Serum reference | Total IgE (kU/L) |
|---|---|
| A | 812 |
| B | 258 |
| C | 45 |
| Healthy | 0 |

Serial dilutions of the sera from 5-fold to 640-fold were prepared and tested following the method in Example 2. The results are shown in Figure 2 and demonstrate that the subjects with allergic disease have elevated levels of sialylated IgE which scales linearly with dilution over the linear working range of the method. Thus, the method is able to be used with complex biological samples and does not require prior purification of IgE.

**Example 5 - Confirmation of specificity for sialylated IgE**

Sera from 13 patients (I - XIII) with respiratory allergy (rinhitis and/or asthma) and sensitized to house dust mites were treated with sialidase A (Agilent) diluted in reaction buffer to 0.5 U/mL during 72 h at 37 °C. The desialylated serum samples, as well as the untreated samples (maintained in the same conditions without enzyme), were diluted 10-fold and tested following the method in Example 2. The results are shown in Table 2.

Table 2: Effect of desialylation with sialidase A enzyme on the result of the method.

| Serum sample | Untreated sample ($A_{450}$ nm) | Desialylated sample ($A_{450}$ nm) | Decrease in Absorbance (%) |
|---|---|---|---|
| I | 1.369 | 0.190 | 86 |
| II | 1.153 | 0.420 | 64 |
| III | 1.134 | 0.459 | 59 |
| IV | 0.900 | 0.056 | 94 |
| V | 0.815 | 0.187 | 77 |
| VI | 0.790 | 0.056 | 93 |
| VII | 0.670 | 0.003 | 100 |
| VIII | 0.592 | 0.099 | 83 |
| IX | 0.580 | 0.237 | 59 |
| X | 0.871 | 0.357 | 59 |
| XI | 0.939 | 0.000 | 100 |
| XII | 0.718 | 0.280 | 61 |
| XIII | 0.804 | 0.010 | 99 |

The data shows that desialylation leads to a decrease in the absorbance of, on average 80 %. This data shows that the method is specific for sialylated IgE.

**Example 6 - Comparison of subjects with and without allergic disease (small sample size)**

Sera from five human subjects were analysed. Subjects 1 and 2 were clinically diagnosed with allergic asthma caused by house dust mites. Subjects 3, 4 and 5 did not suffer from allergic disease and subject 5 also did not have detectable total IgE as determined by ImmunoCAP™ assay. Total IgE (kU/L) are given in Table 3.

Table 3: Clinical information and total IgE for each serum sample

| Serum reference | Clinical info | Total IgE (kU/L) |
|---|---|---|
| 1 | Allergic asthma | 150.0 |
| 2 | Allergic asthma | 42.5 |
| 3 | Healthy | 135.6 |
| 4 | Healthy | 40.7 |
| 5 | Healthy | 0.0 |

Sera were diluted 20-fold into Buffer A and the method according to Example 2 was carried out in triplicate for each sample as well as for a Buffer A control.

The results are shown in Table 4. Table 4:

| Results of sample testing for triplicate assays for sialylated IgE ( $\% CV = 100 \times \frac{\sigma}{\mu}$ ) | | | | | |
|---|---|---|---|---|---|
| **Serum reference** | **A$_{450\ nm}$** | | | **Average A$_{450\ nm}$** | **% CV** |
| 1 | 2.487 | 2.346 | 2.205 | 2.346 | 7 |
| 2 | 1.398 | 1.499 | 1.316 | 1.404 | 7 |
| 3 | 0.229 | 0.226 | 0.271 | 0.242 | 10 |
| 4 | 0.286 | 0.322 | 0.337 | 0.315 | 8 |
| 5 | 0.232 | 0.241 | 0.275 | 0.249 | 9 |
| Control | 0.237 | 0.190 | 0.201 | 0.209 | 12 |

The results for sera subjects with allergic disease and without allergic disease show clear differences with the sera from subjects without allergic disease being very similar to the Buffer A control.

**Example 7 -Comparison of subjects with and without allergic disease (larger sample size)**

Sera or plasma from 435 subjects was obtained. The allergic disease status of the subjects is shown in Table 5.

Table 5: Allergic disease status for 435 subjects

| Allergic disease status | Sample size |
|---|---|
| Respiratory allergy (rhinitis and/or asthma) | 377 |
| Allergy to Hymenoptera venom | 28 |
| No allergic disease | 30 |

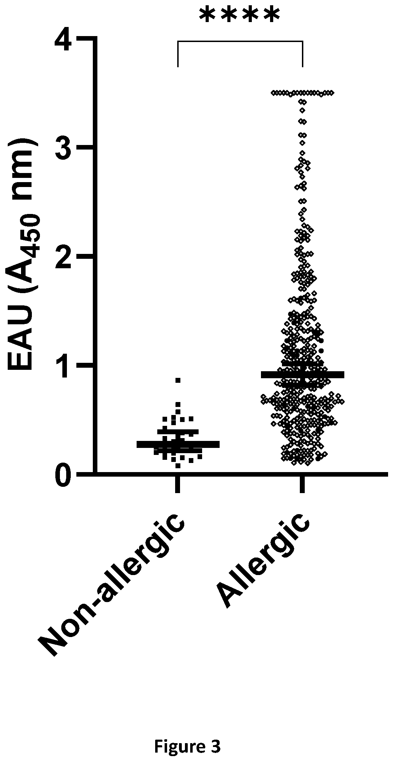

Serum/plasma samples were diluted 20-fold and tested according to the method of Example 2. The results are shown in Figure 3. The data shows a statistically significant difference between the samples from subjects with allergic disease vs those without allergic disease (p < 0.0001 using Mann Whitney test).

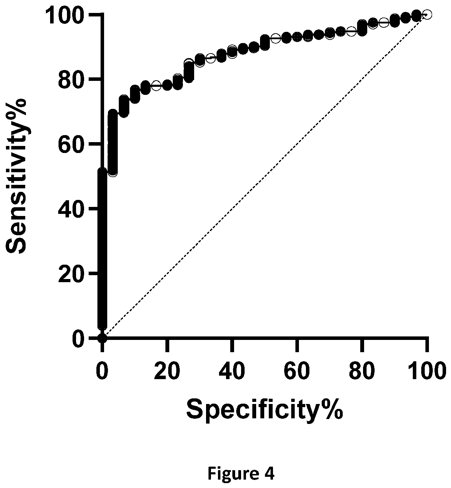

An ROC curve for the results of the method is shown in Figure 4. The area under the ROC curve is 0.878 with a 95 % confidence interval between 0.834 and 0.922 (p < 0.0001) indicating a strong diagnostic test. The ROC curve gives a cut-off A$_{450\ nm}$ of 0.55 to distinguish subjects with allergic disease from those without. This cut-off is the maximum of Youden's index for the test:

$$\text{Sensitivity} = \frac{\text{True positives}}{\text{True positives+False negatives}} = 0.7$$

$$\text{Specificity} = \frac{\text{True negatives}}{\text{True negatives+False positives}} = 0.9$$

$$\text{Positive predictive value} = \frac{\text{True positives}}{\text{True positives+False positives}} = 0.99$$

$$\text{Negative predictive value} = \frac{\text{True negatives}}{\text{True negatives+False negatives}} = 0.21$$

$$\text{Youden's index} = \text{Sensitivity} + \text{Specificity} - 1 = 0.6$$

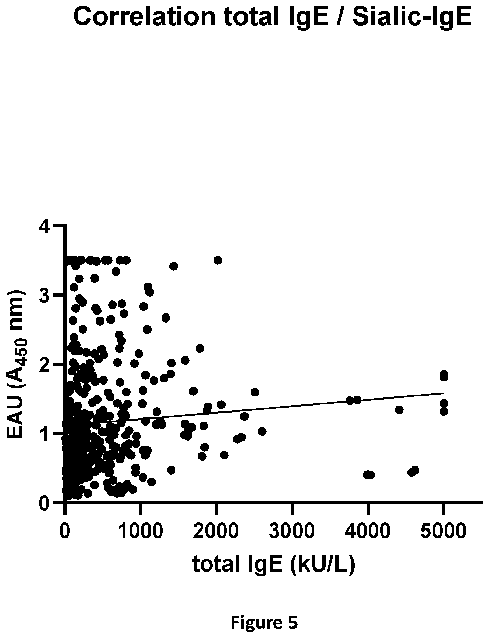

The total IgE content of these 435 samples was also determined and compared to the results from the method (Figure 5). Weak correlation can be seen between the two variables and a Spearman analysis of the samples from subjects with allergic disease suggests that the variables are independent ($r=0.26$, $p < 0.0001$).

**Example 8 - Alternative assay configuration**

Some ELISA assays are known to be reversible, i.e. the surface binding and recognition agents can be switched. In fact, due to the relative scarcity of IgE in blood it would make sense to enrich for IgE first before selecting for sialylated IgE.

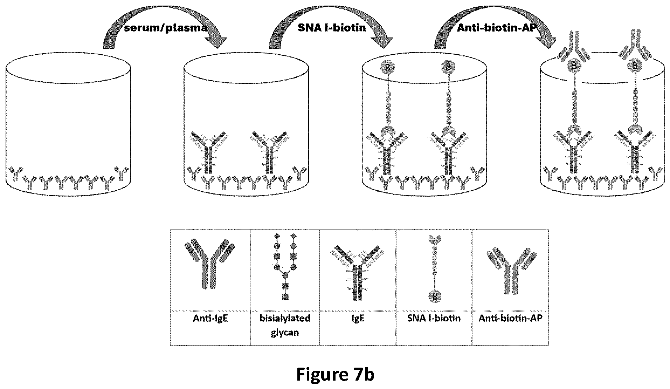

A method was trialled where IgE was immobilised in the wells of plates using immobilised anti-IgE antibodies. Bound sialylated IgE was detected using SNA-1 conjugated to biotin and the biotin was developed using anti-biotin polyclonal antibody conjugated to an alkaline phosphatase. A schematic of this method is shown in Figure 7b.

A Maxisorp™ 96 well ELISA plate (Thermo Scientific™) was coated with anti-IgE monoclonal antibody (clon 4F4, Santa Cruz Biotechnology) at 0.25 $\mu$g mAb/well diluted in 0.01 M PBS (phosphate buffer solution) and the plate was incubated overnight.

The plate was washed with Buffer D (0.1 % (w/v) Tween™ 20 in TBS (Tris 10 mM plus NaCl 150 mM) and then blocked for 1 hour using 100 $\mu$l/well Buffer E (5% (w/v) BSA, 0.1 % (w/v) Tween™ 20 in TBS). The plate was then washed with Buffer D again.

Samples were diluted from 50 to 12,150-fold in Buffer F (1% (w/v) BSA, 0.1 % (w/v) Tween™ 20 in TBS) and added to the plates at 50 $\mu$l/well. Plates were incubated for 2 hours and then washed with Buffer D.

SNA-1 lectin conjugated with biotin (Vector laboratories) was diluted to 0.4 $\mu$g/ml in Buffer G (1% (w/v) BSA, 0.1% (w/v) Tween™ 20, 0.1 M calcium chloride, 0.1 M magnesium chloride in TBS) and 50 $\mu$l/well was added. The plates were incubated for 1 hour and then washed with Buffer D.

50 $\mu$l/well of anti-biotin polyclonal antibody conjugated with Alkaline phosphatase (Thermo Fischer) diluted 3000-fold in Buffer F was added and the plates were incubated for 1 hour and then washed with Buffer D.

p-Nitrophenyl Phosphate (pNPP) substrate (Merck) was added at 50 $\mu$l/well and incubated for 30 minutes. The reaction was then stopped by adding 50 $\mu$l/well of 2N NaOH.

Absorbance of each well at 415 nm was measured using a plate reader.

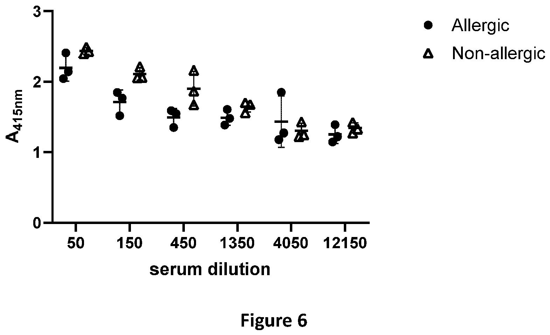

The results are shown in Figure 6 and demonstrate that the results for samples from subjects with and without allergic disease are largely indistinguishable. Thus, this configuration of the assay is not suitable and it is surprising that the method as exemplified in Example 2 and illustrated in Figure 7a shows such clear discrimination between healthy subjects and those with allergic disease.

**References**

1. Ansotegui IJ, Melioli G, Canonica GW, Caraballo L, Villa E, Ebisawa M, et al. IgE allergy diagnostics and other relevant tests in allergy, a World Allergy Organization position paper. World Allergy Organ J. 2020;13(2):100080.

2. Tatar EC, Surenoglu UA, Saylam G, Isik E, Ozdek A, Korkmaz H. Is there any correlation between the results of skin-

EP 4 621 411 A1

prick test and the severity of symptoms in allergic rhinitis? Am J Rhinol Allergy. 2012;26(1):e37-9.

3. Plomp R, Hensbergen PJ, Rombouts Y, Zauner G, Dragan I, Koeleman CA, et al. Site-specific N-glycosylation analysis of human immunoglobulin e. J Proteome Res. 2014;13(2):536-46.

4. Shade KT, Platzer B, Washburn N, Mani V, Bartsch YC, Conroy M, et al. A single glycan on IgE is indispensable for initiation of anaphylaxis. J Exp Med. 2015;212(4):457-67.

5. Shade KC, Conroy ME, Washburn N, Kitaoka M, Huynh DJ, Laprise E, et al. Sialylation of immunoglobulin E is a determinant of allergic pathogenicity. Nature. 2020;582(7811):265-70.

6. Vattepu R, Sneed SL, Anthony RM. Sialylation as an Important Regulator of Antibody Function. Front Immunol. 2022;13:818736.

7. Wu J, Zhu J, Yin H, Buckanovich RJ, Lubman DM. Analysis of Glycan Variation on Glycoproteins from Serum by the Reverse Lectin-Based ELISA Assay. J. Proteome Res. 2014;13:2197-2204.

8. Thorpe S, Heath A, Fox B, Patel D, Egner W. The 3rd International Standard for serum IgE: International collaborative study to evaluate a candidate preparation. Clinical chemistry and laboratory medicine : CCLM / FESCC. 2014;52.

**Claims**

1. A method of quantifying sialylated IgE in a test sample, the method comprising:

   immobilising sialylated IgE in the test sample via a capture probe which is specific for sialylated glycoproteins, wherein the capture probe is immobilised; and
   using an antibody-based detection technique to quantify sialylated IgE.

2. The method according to claim 1, wherein the capture probe is SNA-1 lectin.

3. The method according to claim 1 or claim 2, wherein the capture probe is immobilised to the surface of a well of a plate.

4. The method according to any one of the preceding claims, wherein the antibody-based detection technique comprises a colorimetric assay.

5. The method according to any one of the preceding claims, wherein the antibody-based detection technique comprises the use of an IgE recognising antibody, optionally wherein the IgE recognising antibody is conjugated to a horseradish peroxidase enzyme.

6. The method according to claim 1, wherein the method comprises:

   a) immobilizing SNA-1 lectin in the well of an ELISA plate;
   b) adding a test sample and allowing any sialylated glycoproteins to bind to the immobilised SNA-1 lectin;
   c) adding an anti-IgE antibody conjugated to horseradish peroxidase and allowing the antibody to bind to any bound sialylated IgE;
   d) adding a developing solution comprising a chromophore precursor and hydrogen peroxide, allowing oxidation of the chromophore precursor to a chromophore to occur, and detecting the production of the chromophore using spectrophotometry.

7. The method according to claim 6, wherein between step a) and step b) is a blocking step comprising addition of an inert substance which saturates the ELISA plate.

8. The method according to claim 7 or claim 8 wherein the chromophore precursor is diaminobenzidine.

9. The method according to any one of the preceding claims, wherein the test sample is blood serum or plasma from a subject, optionally wherein the blood serum or plasma is from a human subject or a non-human subject.

10. A diagnostic method for diagnosing allergy in a subject, the diagnostic method comprising using the method of claim 9 and diagnosing the subject with allergy based on the quantity of sialylated IgE in the test sample.

11. The diagnostic method according to claim 10, wherein the test sample is blood serum from a human subject and the subject is diagnosed with allergy if the test sample comprises sufficient sialylated IgE such that, when tested according to a method comprising:

> a) immobilizing SNA-1 lectin in a well of a standard 96-well plate;
> b) diluting a test sample 20-fold into an inert buffer (preferably phosphate buffered saline);
> c) adding 100 μL of the diluted test sample to the well and allowing any sialylated glycoproteins to bind to the immobilised SNA-1 lectin;
> d) adding an anti-IgE antibody conjugated to horseradish peroxidase and allowing the antibody to bind to any bound sialylated IgE; and
> e) adding a developing solution comprising diaminobenzidine and hydrogen peroxide, allowing oxidation of the chromophore precursor to a chromophore to occur,
> f) halting oxidation though addition of a peroxidase inhibitor such that the total solution volume is 200 μL; and
> g) detecting the production of the chromophore using spectrophotometry;

> the chromophore elicits an absorbance of at least 0.55 at 450 nm.

12. The diagnostic method according to claim 10 or claim 11 wherein if the subject is diagnosed with allergy, the diagnostic method further comprises a suitable clinical or *in vitro* method to determine if a subject is allergic to any allergy source.

13. A method of treatment of allergy or allergic disease, the method comprising the steps of:

> diagnosis of allergy according to any one of claims 10-12; and
> treatment by immunotherapy.

14. A kit comprising:

> a capture probe which is specific for sialylated glycoproteins wherein the capture probe is either immobilised or is readily capable of immobilisation; and
> an IgE recognising antibody;
> optionally wherein the capture probe is SNA-1 lectin.

15. Use of a kit according to claim 14 to quantify sialylated IgE in a test sample.

## ELISA sialic-IgE

**Figure 1**

## Sialic-IgE in serum

**Figure 2**

Figure 3

Figure 4

Correlation total IgE / Sialic-IgE

Figure 5

**Figure 6**

**Figure 7a**

| Anti-IgE | bisialylated glycan | IgE | SNA I-biotin | Anti-biotin-AP |

**Figure 7b**

Europäisches
Patentamt

European
Patent Office

Office européen
des brevets

# EUROPEAN SEARCH REPORT

**Application Number**

EP 24 38 2317

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WU JING ET AL: "Analysis of Glycan Variation on Glycoproteins from Serum by the Reverse Lectin-Based ELISA Assay", JOURNAL OF PROTEOME RESEARCH, vol. 13, no. 4, 4 April 2014 (2014-04-04), pages 2197-2204, XP055894761, ISSN: 1535-3893, DOI: 10.1021/pr401061c Retrieved from the Internet: URL:https://www.ncbi.nlm.nih.gov/pmc/articles/PMC3993964/pdf/pr401061c.pdf> | 1-9 | INV. G01N33/68 |
| Y | * abstract, pg 2198, left col, last par; right col last par-pg 2199, left col, first par * | 10-15 | |
| Y | SHADE KAI-TING C ET AL: "Sialylation of immunoglobulin E is a determinant of allergic pathogenicity", NATURE,, vol. 582, no. 7811, 20 May 2020 (2020-05-20), pages 265-270, XP037183254, DOI: 10.1038/S41586-020-2311-Z [retrieved on 2020-05-20] * abstract, pg 270, left col, last par * | 10-15 | |
| X | US 2022/105179 A1 (ANTHONY ROBERT M [US] ET AL) 7 April 2022 (2022-04-07) | 14,15 | TECHNICAL FIELDS SEARCHED (IPC) |
| Y | * abstract, pg 270, left col, last par * | 10-13 | G01N |
| A | US 2017/122940 A1 (KANEKO TOMONORI [JP] ET AL) 4 May 2017 (2017-05-04) * the whole document * | 1-15 | |

The present search report has been drawn up for all claims

| Place of search | Date of completion of the search | Examiner |
|---|---|---|
| Munich | 26 September 2024 | Motrescu-Hateley, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                                             
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 38 2317

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2024

| Patent document cited in search report | Publication date | Patent family member(s) | Publication date |
|---|---|---|---|
| US 2022105179 A1 | 07-04-2022 | US 2022105179 A1 | 07-04-2022 |
| | | WO 2020172072 A1 | 27-08-2020 |
| US 2017122940 A1 | 04-05-2017 | EP 3159694 A1 | 26-04-2017 |
| | | JP 6520940 B2 | 29-05-2019 |
| | | JP WO2015194350 A1 | 20-04-2017 |
| | | US 2017122940 A1 | 04-05-2017 |
| | | WO 2015194350 A1 | 23-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ANSOTEGUI IJ** ; **MELIOLI G** ; **CANONICA GW** ; **CARABALLO L** ; **VILLA E** ; **EBISAWA M et al.** IgE allergy diagnostics and other relevant tests in allergy, a World Allergy Organization position paper. *World Allergy Organ J.*, 2020, vol. 13 (2), 100080
- **TATAR EC** ; **SURENOGLU UA** ; **SAYLAM G** ; **ISIK E** ; **OZDEK A** ; **KORKMAZ H**. Is there any correlation between the results of skin-prick test and the severity of symptoms in allergic rhinitis?. *Am J Rhinol Allergy*, 2012, vol. 26 (1), e37-9
- **PLOMP R** ; **HENSBERGEN PJ** ; **ROMBOUTS Y** ; **ZAUNER G** ; **DRAGAN I** ; **KOELEMAN CA et al.** Site-specific N-glycosylation analysis of human immunoglobulin e.. *J Proteome Res.*, 2014, vol. 13 (2), 536-46
- **SHADE KT** ; **PLATZER B** ; **WASHBURN N** ; **MANI V** ; **BARTSCH YC** ; **CONROY M et al.** A single glycan on IgE is indispensable for initiation of anaphylaxis.. *J Exp Med.*, 2015, vol. 212 (4), 457-67

- **SHADE KC** ; **CONROY ME** ; **WASHBURN N** ; **KITAOKA M** ; **HUYNH DJ** ; **LAPRISE E et al.** Sialylation of immunoglobulin E is a determinant of allergic pathogenicity.. *Nature*, 2020, vol. 582 (7811), 265-70
- **VATTEPU R** ; **SNEED SL** ; **ANTHONY RM**. Sialylation as an Important Regulator of Antibody Function. *Front Immunol.*, 2022, vol. 13, 818736
- **WU J** ; **ZHU J** ; **YIN H** ; **BUCKANOVICH RJ** ; **LUBMAN DM**. Analysis of Glycan Variation on Glycoproteins from Serum by the Reverse Lectin-Based ELISA Assay.. *J. Proteome Res.*, 2014, vol. 13, 2197-2204
- **THORPE S** ; **HEATH A** ; **FOX B** ; **PATEL D** ; **EGNER W.** The 3rd International Standard for serum IgE: International collaborative study to evaluate a candidate preparation.. *Clinical chemistry and laboratory medicine : CCLM / FESCC*, 2014, vol. 52